# EUROPÄISCHE PATENTSCHRIFT

(11) **EP 1 392 652 B1**
(45) Veröffentlichungstag und Bekanntmachung des Hinweises auf die Patenterteilung: **22.03.2006**
(21) Anmeldenummer: 02737988.2
(22) Anmeldetag: 24.04.2002
(51) Int. Cl.: C07D 213/61, C07D 213/36, C07D 277/32, C07D 277/28, C07D 307/14

(54) **VERFAHREN ZUR HERSTELLUNG VON 1,3-DISUBSTITUIERTEN 2-NITROGUANIDINEN**
METHOD FOR PRODUCING 1,3-DISUBSTITUTED 2-NITROGUANIDINES
PROCEDE DE PRODUCTION DE 2-NITROGUANIDINES 1,3-DISUSBTITUEES

(30) Priorität: 03.05.2001 DE 10121652
(43) Veröffentlichungstag der Anmeldung: 03.03.2004
(73) Patentinhaber: Bayer CropScience AG, 40789 Monheim (DE)
(72) Erfinder: STÖLTING, Jörn, 51061 Köln (DE); VAN LAAK, Kai, 38302 Wolfenbüttel (DE); SIRGES, Wolfram, 40597 Düsseldorf (DE); HEYN, Armin, 51467 Bergisch Gladbach (DE); TASCHNER, Torsten, 50823 Köln (DE)
(86) Internationale Anmeldenummer: PCT/EP2002/004474
(87) Internationale Veröffentlichungsnummer: WO 2002/090331

(56) Entgegenhaltungen:
- EP-A- 0 483 062
- WO-A-99/09009
- WARNHOFF, H. ET AL.: "Photodegradation of Imidacloprid" J. AGRIC. FOOD CHEM., Bd. 47, 1999, Seiten 1730-1734, XP002212770
- KAGABU, S. ET AL.: "5-Azidoimidacloprid and an Acyclic Analogue as Candidate Photoaffinity Probes for Mammalian and Insect Nicotinic Acetylcholine Receptors" J. MED. CHEM., Bd. 43, 2000, Seiten 5003-5009, XP002212771
- MAIENFISCH, P. ET AL.: "A novel method for the preparation of N,N'-disubstituted N''-nitroguanidines, including a practical synthesis of the neonicotinoid clothianidin" TETRAHEDRON LETTERS, Bd. 41, 2000, Seiten 7187-7191, XP002212772

## Beschreibung

Die vorliegende Erfindung betrifft ein neues Verfahren zur Herstellung von 1,3-disubstituierten 2-Nitroguanidinen.

Aus EP-A-0 483 062 ist ein Verfahren zur Herstellung von 1,3-disubstituierten 2-Nitroguanidinen bekannt. Sie werden erhalten durch Hydrolyse entsprechender 2-Nitroimino-1,3,5-triazacyclohexanderivate. Die Hydrolyse wird bevorzugt in Gegenwart starker Mineralsäuren oder organischer Säuren durchgeführt.

Nachteilig bei diesem Verfahren sind die langen Reaktionszeiten und das Entstehen von Nebenprodukten, die eine aufwändige Reinigung der gewünschten Endprodukte erforderlich machen.

Darüber hinaus müssen beim Arbeiten in Gegenwart wässriger starker Säuren bekanntlich Maßnahmen zum Schutz beispielsweise der Reaktoren gegen Korrosion getroffen werden.

JP 3291267, JP 10067766, JP 10147580 und WO 99/09009 betreffen ähnliche Verfahren.

Aufgabe der vorliegenden Erfindung war es, ein verbessertes Verfahren zur Herstellung von 1,3-disubstituierten 2-Nitroguanidinen bereitzustellen.

Gegenstand der vorliegenden Erfindung ist ein Verfahren zur Herstellung von Verbindungen der Formel (I) 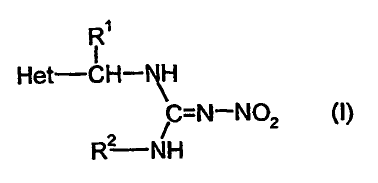 worin
- R¹: für Wasserstoff oder C₁-C₄-Alkyl steht,
- R²: für Wasserstoff, C₁-C₆-Alkyl, C₃-C₆-Cycloalkyl oder einen Rest -CH₂R³ steht,
- R³: für C₂-C₅-Alkenyl, C₂-C₅-Alkinyl, Phenyl, Cyanophenyl, Nitrophenyl, Halogenphenyl mit 1 bis 3 Halogenatomen; durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl; durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃-Alkoxy, C₂-C₃₋Halogenalkenyl mit 1 bis 5 Halogenatomen, C₂-C₃-Halogenalkinyl mit 1 bis 3 Halogenatomen, C₁-C₃-Halogenalkoxy mit 1 bis 7 Halogenatomen, C₁-C₃₋Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Halogen, Cyano oder Nitro substituiertes 5-Thiazolyl; oder durch ein bis vier (bevorzugt ein oder zwei) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 5 Halogenatomen, C₂₋C₃-Halogenalkinyl mit 1 bis 3 Halogenatomen, C₁-C₃-Halogenalkoxy mit 1 bis 7 Halogenatomen, C₁-C₃ Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃-Alkoxy oder Halogen substituiertes 3-Pyridyl steht,
- Het: für einen unsubstituierten oder substituierten aromatischen oder nichtaromatischen, monocyclischen oder bicyclischen heterocyclischen Rest bevorzugt aus der Reihe 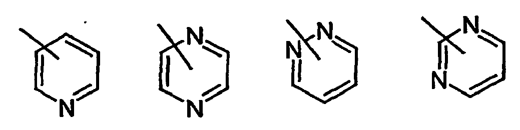 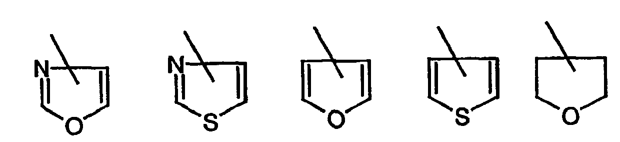 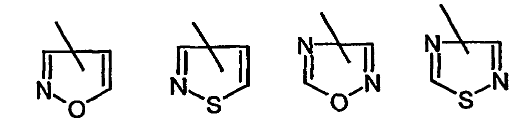 steht, der ein oder zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 5 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃₋Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃₋Alkoxy und Halogen enthalten kann,
dadurch gekennzeichnet, dass man eine Verbindung der Formel (II) 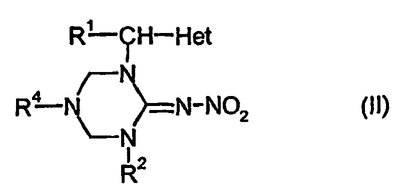 worin
- R¹, R² und Het: die oben angegebenen Bedeutungen haben und
- R⁴: für jeweils unsubstituiertes oder substituiertes C₁-C₁₀-Alkyl, C₃-C₆-Cycloalkyl, Phenyl, Arylalkyl oder Heterocyclylmethyl steht, wobei Heterocyclyl für einen ungesättigten oder gesättigten 5- oder 6-gliedrigen Heterocyclus mit einem oder mehreren Heteroatomen aus der Reihe Stickstoff, Sauerstoff und Schwefel wie z.B. Furan, Tetrahydrofuran, Thiophen oder Pyridin steht,
in Gegenwart eines Nitrils mit 3 bis 5 Kohlenstoffatomen mit wasserfreiem Halogenwasserstoff umsetzt.

Die Verbindungen der Formel (I) können auch als Doppelbindungsisomere bezüglich der -N=C(2)-Bindung und in ihren tautomeren Formen (Formeln Ia, Ib) auftreten:

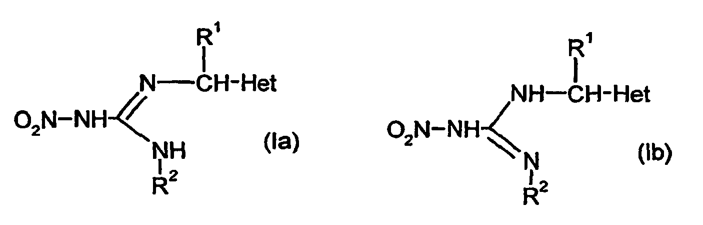

Formel (I) ist demnach so zu verstehen, dass sie auch die entsprechenden Doppelbindungsisomeren und die Formeln (Ia) und (Ib) einschließt

Überraschenderweise liefert das erfindungsgemäße Verfahren selektiv und in hohen Ausbeuten die Endprodukte der Formel (I) nach kurzer Reaktionszeit unter milden Reaktionsbedingungen in reiner Form.

Ein besonderer Vorteil des erfindungsgemäßen Verfahrens liegt in der Verwendung des Nitrils. Denn beim Abkühlen der Reaktionsmischung kristallisiert das Endprodukt direkt aus und kann so in einfacher Weise isoliert werden.
Verwendet man beispielsweise 1-(2-Chlorthiazol-5-ylmethyl)-2-nitro-imino-5-benzyl-3-methyl-1,3,5-triazacyclohexan als Ausgangsstoff und wasserfreien Chlorwasserstoff sowie Butyronitril, so kann der Verlauf des erfindungsgemäßen Verfahrens durch das folgende Formelschema wiedergegeben werden:

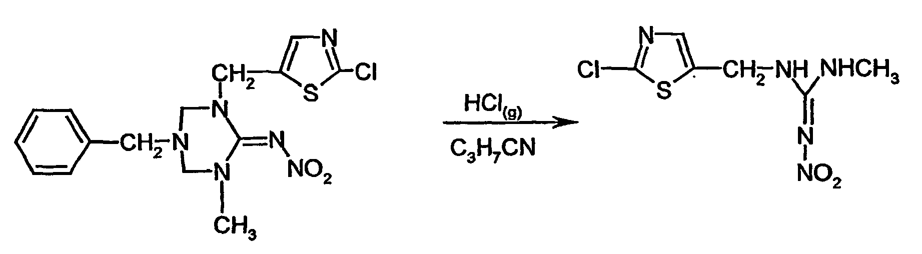

Die als Ausgangsstoffe für das erfindungsgemäße Verfahren benötigten Verbindungen sind durch die Formel (II) allgemein definiert.

Bevorzugte Substituenten bzw. Bereiche der in den oben und nachstehend erwähnten Formeln aufgeführten Reste werden im Folgenden erläutert:
- R¹: steht bevorzugt für Wasserstoff, Methyl, Ethyl, n- oder i-Propyl.
- R²: steht bevorzugt für Wasserstoff, Methyl, Ethyl, n-Propyl, i-Propyl oder n-Butyl, Cyclopropyl, Cyclopentyl, Cyclohexyl oder für einen Rest -CH₂R³.
- R³: steht bevorzugt für C₂-C₅-Alkenyl, C₂-C₅-Alkinyl, Phenyl, Cyanophenyl, Nitrophenyl, Halogenphenyl mit 1 bis 3 Halogenatomen; durch C₁-C₃-Alkyl, C₁-C₃-Halogenalkyl mit 1 bis 7 Halogenatomen, C₁-C₃-Alkoxy oder C₁-C₃₋Halogenalkoxy mit 1 bis 7 Halogenatomen substituiertes Phenyl; durch ein bis zwei (bevorzugt einen) Substituenten aus der Gruppe C₁-C₃-Alkyl, C₁₋C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃₋Alkoxy, C₂-C₃-Halogenalkenyl mit 1 bis 5 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Halogen, Cyano oder Nitro substituiertes 5-Thiazolyl; oder durch ein bis zwei (bevorzugt einen) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl mit 1 bis 5 Halogenatomen, C₂-C₃-Halogenalkinyl mit 1 bis 3 Halogenatomen, C₁-C₃₋Halogenalkoxy mit 1 bis 7 Halogenatomen, C₁-C₃ Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃-Alkoxy oder Halogen substituiertes 3-Pyridyl.
- R⁴: steht bevorzugt für unsubstituiertes oder durch 1 bis 6 Reste aus der Gruppe Halogen, Hydroxy, C₁-C₄-Alkoxy, C₁-C₄-Halogenalkoxy mit 1 bis 9 Halogenatomen, Di-(C₁-C₄-alkyl)-amino oder C₁-C₅-Alkoxycarbonyl substituiertes C₁-C₁₀-Alkyl; für unsubstituiertes oder durch 1 bis 4 Reste aus der Reihe C₁-C₄-Alkyl oder Halogen substituiertes C₃-C₆-Cycloalkyl; für jeweils unsubstituiertes oder jeweils durch 1 bis 3 Ringsubstituenten aus der Gruppe Halogen, C₁-C₄-Alkyl, C₁-C₄-Halogenalkyl mit 1 bis 9 Halogenatomen, C₁₋C₄-Alkoxy, C₁-C₄-Hatogenalkoxy mit 1 bis 9 Halogenatomen, C₁-C₄₋Alkylthio, Nitro oder Cyano substituiertes Phenyl, Benzyl oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht.
- Het: steht bevorzugt für einen unsubstituierten oder einfach oder zweifach (bevorzugt einfach) substituierten heterocyclischen Rest aus der Reihe 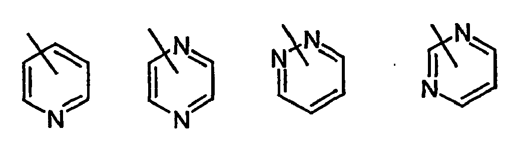 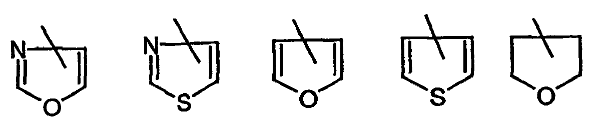 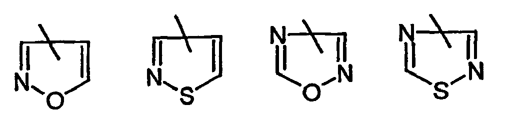
insbesondere für 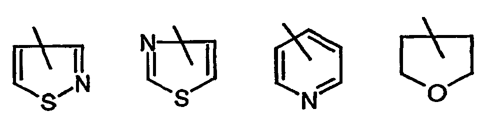 wobei die Substituenten aus der Reihe Fluor, Chlor, Brom, Methyl, Ethyl, Methoxy und Ethoxy ausgewählt sind.
- R¹: steht besonders bevorzugt für Wasserstoff, Methyl oder Ethyl.
- R²: steht besonders bevorzugt für Wasserstoff, Methyl, Ethyl, n-Propyl, i-Propyl, n-Butyl, Cyclopropyl, Cyclopentyl, Cyclohexyl oder für einen Rest -CH₂R³.
- R³: steht besonders bevorzugt für C₂-C₃-Alkenyl, C₂-C₃-Alkinyl, Phenyl, Cyanophenyl, Nitrophenyl, Halogenphenyl mit 1 bis 3 Halogenatomen; durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl; jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃₋Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano oder Nitro substituiertes 5-Thiazolyl oder 3-Pyridyl.
- R⁴: steht besonders bevorzugt für C₁-C₁₀-Alkyl; für durch 1 bis 6 Reste aus der Gruppe Halogen, C₁-C₄-Alkoxy und C₁-C₄-Halogenalkoxy mit 1 bis 9 Halogenatomen substituiertes C₁-C₈-Alkyl; für unsubstituiertes oder durch 1 oder 2 Reste aus der Reihe Methyl, Ethyl, Fluor und Chlor substituiertes C₃-C₆₋Cycloalkyl; für unsubstituiertes oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl, Benzyl oder Heterocyclylmethyl, wobei Heterocyclyl für einen ungesättigten oder gesättigten 5- oder 6-gliedrigen Heterocyclus mit einem Heteroatom aus der Reihe Stickstoff, Sauerstoff und Schwefel wie z.B. Furan, Tetrahydrofuran, Thiophen oder Pyridin steht.
- Het: steht besonders bevorzugt für jeweils unsubstituiertes oder einfach oder zweifach (insbesondere einfach) substituiertes Thiazolyl, Pyridyl oder Tetrahydrofuranyl, wobei die Substituenten aus der Reihe Fluor, Chlor, Methyl und Methoxy ausgewählt sind.
- R¹: steht ganz besonders bevorzugt für Wasserstoff, Methyl oder Ethyl, hervorgehoben für Wasserstoff
- R²: steht ganz besonders bevorzugt für Wasserstoff, Methyl, Ethyl, n-Propyl, i-Propyl, n-Butyl, Cyclopropyl, Cyclopentyl, Allyl, Propargyl, Benzyl, p-Chlorbenzyl, 3-Pyridylmethyl oder 6-Chlor-3-pyridylmethyl.
- R4: steht ganz besonders bevorzugt für Methyl, Ethyl, n-Propyl, Cyclopropyl, Cyclopentyl, Cyclohexyl, Phenyl, Benzyl oder Furfuryl.
- Het: steht ganz besonders bevorzugt für einen der Reste

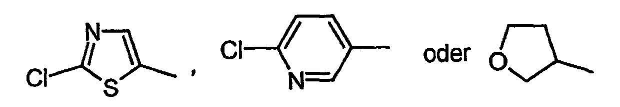

Bei allen allgemeinen Restedefinitionen sowie in den Vorzugsbereichen stehen Halogen(atome) bevorzugt für F, Cl, Br, I, insbesondere für F, Cl, Br und hervorgehoben für F, Cl.

Besonders hervorgehoben seien als Ausgangsstoffe für das erfindungsgemäße Verfahren Verbindungen der Formeln (IIa), (IIb) und (IIc) 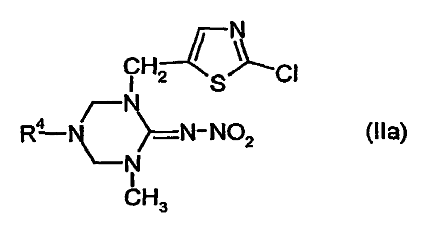 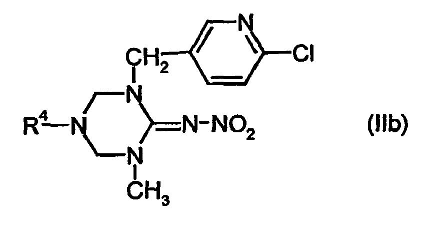 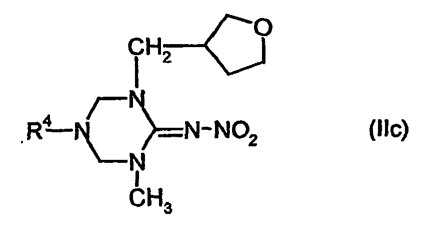 in welchen
- R⁴: für Methyl, Ethyl, Cyclopropyl, Cyclopentyl, Benzyl oder Furfuryl steht, wobei unter diesen wiederum Methyl, Benzyl und Furfuryl bevorzugt sind.

Als Endprodukte des erfindungsgemäßen Verfahrens erhält man bei Verwendung einer Verbindung der Formel (IIa) die folgende Verbindung 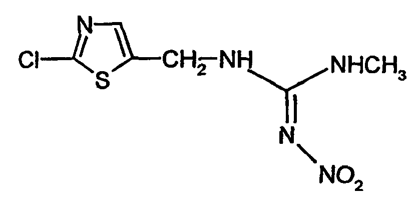 bei Verwendung einer Verbindung der Formel (IIb) die folgende Verbindung 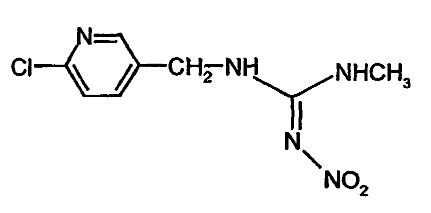 und bei Verwendung einer Verbindung der Formel (IIc) die folgende Verbindung

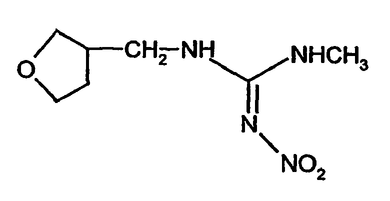

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Unter der Bezeichnung Alkyl sind dabei auch die verzweigten Isomere, z.B. t-Butyl für C₄-Alkyl, zu verstehen.

Bevorzugt werden diejenigen Verbindungen der Formel (II) in das erfindungsgemäße Verfahren eingesetzt, in welchen eine Kombination der vorstehend als bevorzugt aufgeführten Bedeutungen vorliegt.

Besonders bevorzugt werden diejenigen Verbindungen der Formel (II) in das erfindungsgemäße Verfahren eingesetzt, in welchen eine Kombination der vorstehend als besonders bevorzugt aufgeführten Bedeutungen vorliegt.

Ganz besonders bevorzugt werden diejenigen Verbindungen der Formel (II) in das erfindungsgemäße Verfahren eingesetzt, in welchen eine Kombination der vorstehend als ganz besonders bevorzugt aufgeführten Bedeutungen vorliegt.

Die Ausgangsstoffe der Formel (II) sind bekannt oder können nach bekannten Verfahren hergestellt werden (vgl. EP-A-0 483 062, JP 3 291 267, EP-A-0 483 055, EP-A-0 428 941, EP-A-0 386 565, WO 98/42690).

Das erfindungsgemäße Verfahren wird in Gegenwart eines Nitrils mit 3 bis 5 Kohlenstoffatomen durchgeführt.

Als Nitrile eignen sich aliphatische Mono- und Dinitrile mit 3 bis 5 Kohlenstoffatomen. Vorzugsweise verwendbar sind Propionitril, Butyronitril, Valeronitril, Malonsäuredinitril (Malononitril), Bernsteinsäuredinitril (Succinonitril), Glutarsäuredinitril (Glutaronitril). Besonders bevorzugt verwendet man Propionitril oder Butyronitril, ganz besonders bevorzugt Butyronitril.

Es ist auch möglich, Gemische der genannten Nitrile einzusetzen.

Das erfindungsgemäße Verfahren wird bei Temperaturen zwischen -10°C und 200°C, vorzugsweise zwischen 20°C und 150°C, besonders bevorzugt zwischen 40°C und 80°C durchgeführt.

Das erfindungsgemäße Verfahren wird im allgemeinen unter Normaldruck durchgeführt. Es kann aber gegebenenfalls auch unter erhöhtem Druck gearbeitet werden.

Im erfindungsgemäßen Verfahren wird wasserfreier Halogenwasserstoff als Reagenz eingesetzt. Vorzugsweise verwendbar sind Chlorwasserstoff, Bromwasserstoff oder Iodwasserstoff, besonders bevorzugt Chlorwasserstoff oder Bromwasserstoff, ganz besonders bevorzugt Chlorwasserstoff. Der wasserfreie Halogenwasserstoff wird vorzugsweise gasförmig eingesetzt.

Der Halogenwasserstoff wird im allgemeinen in einem molaren Verhältnis von 0,5:1 bis 10:1, vorzugsweise 1:1 bis 6:1, bezogen auf die Ausgangsverbindung der Formel (II), eingesetzt.

Im allgemeinen wird die Umsetzung so durchgeführt, dass man den Ausgangsstoff der Formel (II) in einem Nitril auf die gewünschte Temperatur bringt und den Halogenwasserstoff im Verlauf der Reaktion sukzessive zudosiert.

Zur Aufarbeitung wird nach dem Abkühlen gegebenenfalls mit Wasser und/oder mit Natronlauge versetzt und das Endprodukt gegebenenfalls nach Einengen des Gemischs beispielsweise durch Filtration oder Extraktion isoliert.

Die erfindungsgemäß hergestellten Verbindungen der Formel (I) sind wertvolle Wirkstoffe in der Schädlingsbekämpfung. Insbesondere sind die Verbindungen der Formel (I) geeignet zur Bekämpfung von Insekten und Spinnentieren, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen (siehe z.B. EP-A-0 376 279, EP-A-0 375 907, EP-A-0 383 091).

### Herstellungsbeispiele

Herstellung von 1-(2-Chlorthiazol-5-ylmethyl)-2-nitro-3-methylguanidin

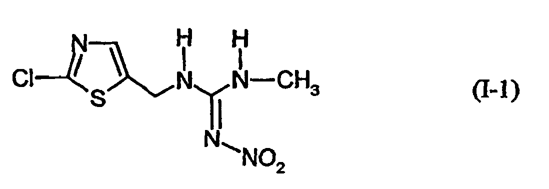

### Beispiel 1

22.1 g (0.05 mol) 1-(2-Chlorthiazol-5-ylmethyl)-2-nitro-imino-5-benzyl-3-methyl-1,3,5-triazacyclohexan (86.1%ig nach ISTD) 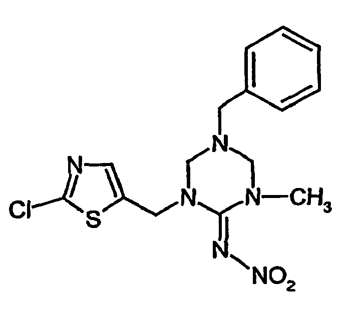 werden in 75 ml Butyronitril vorgelegt, und bei 55 bis 60°C werden innerhalb von 20 min 7.9 g (0.22 mol) gasförmiger Chlorwasserstoff unter Rühren in die Suspension eingeleitet. Man lässt noch 1 h bei 55 bis 60°C nachrühren, kühlt dann auf 0 bis -5°C ab und setzt bei dieser Temperatur 50 ml Eiswasser zu. Nach gutem Durchrühren stellt man die Suspension bei 0 bis -5°C durch Zugabe verdünnter Natronlauge auf pH = 8 ein, rührt 5 min nach, saugt dann ab und wäscht den Niederschlag mit ca. 150 ml Wasser.

Nach dem Trocknen erhält man einen nahezu weißen Feststoff.

| | |
|---|---|
| Ausbeute: | 12.1 g (91.2% d. Th., 94.7%ige Reinheit nach HPLC und 94.1 % Gehalt nach ISTD) |
| ¹H-NMR (DMSO): | δ = 2.80 (3H), 4.50 (2H), 7.60 (1H), 7.94 (breit, 1H), 9.17 (breit, 1 H) ppm. |
| LC/MS: | 250 [M + H]+, 208, 120 (Elektrospray, positiv-Modus). |

### Beispiel 2

547 g (1.2 mol) 1-(2-Chlorthiazol-5-ylmethyl)-2-nitro-imino-5-benzyl-3-methyl-1,3,5-triazacyclohexan (83.4%ig nach ISTD) werden bei Raumtemperatur in 1145 g Butyronitril vorgelegt, es wird auf 60°C aufgeheizt, und bei dieser Temperatur werden innerhalb von 60 min 181 g (4.9 mol) gasförmiger Chlorwasserstoff unter Rühren auf die Suspension aufgeleitet. Man lässt noch 1 h bei 60°C nachrühren und setzt dann bei dieser Temperatur 1080 ml Wasser zu. Die Temperatur fällt auf 50°C, der Feststoff geht vollständig in Lösung, und es bilden sich zwei Phasen. Die Mischung wird auf 20°C abgekühlt, und man stellt durch Zugabe konzentrierter Natronlauge unter Rühren einen pH-Wert von 6 ein. Die entstandene Suspension wird auf 0°C gekühlt, abgesaugt und in einer Verdrängungswäsche mit Wasser (20°C) gewaschen.

Nach dem Trocknen bei 50°C im Vakuumtrockenschrank erhält man einen nahezu weißen Feststoff.

Ausbeute: 275 g (91.1% d. Th., 99. 1 % Gehalt nach ISTD)

### Beispiel 3

22 g (0.05 mol) 1-(2-Chlorthiazol-5-yhnethyl)-2-nitro-imino-5-benzyl-3-methyl-1,3,5-triazacyclohexan (86.6%ig nach ISTD) werden in 75 ml Propionitril vorgelegt, und bei 55 bis 60°C werden innerhalb von 20 min 8.6 g (0.235 mol) gasförmiger Chlorwasserstoff unter Rühren in die Suspension eingeleitet. Man lässt noch 1 h bei 55 bis 60°C nachrühren, kühlt dann auf 0 bis -5°C ab und setzt bei dieser Temperatur 50 ml Eiswasser zu. Nach gutem Durchrühren stellt man die Suspension bei 0 bis -5°C durch Zugabe verdünnter Natronlauge auf pH = 8 ein, rührt 2 h nach, saugt dann ab und wäscht den Niederschlag mit ca. 120 ml Wasser.

Nach dem Trocknen erhält man einen nahezu weißen Feststoff.

| | |
|---|---|
| Ausbeute: | 11.36 g (88.8% d. Th., 98.4%ige Reinheit nach HPLC und 97.6% Gehalt nach ISTD) |

### Beispiel 4

2 g (0.005 mol) 1-(2-Chlorthiazol-5-yhmethyl)-2-nitro-imino-5-benzyl-3-methyl-1,3,5-triazacyclohexan (95.7%ig nach ISTD) werden in 25 ml Butyronitril vorgelegt, und bei 0 bis 5°C wird gasförmiger Chlorwasserstoff bis zur Sättigung unter Rühren in die Suspension eingeleitet. Unter leichter Exothermie bildet sich sofort eine klare Lösung. Man erwärmt auf 80°C, wobei Chlorwasserstoff entweicht, und nach 2 h Rühren bei dieser Temperatur hat sich eine Suspension gebildet. Man kühlt dann auf 0 bis -5°C, saugt ab und erhält einen nahezu weißen Feststoff.
Ausbeute: 1.42 g (95.5% d. Th., 84 % Gehalt nach ISTD)

### Vergleichsbeispiel (nicht erfindungsgemäß)

2 g (0.005 mol) 1-(2-Chlorthiazol-5-ylmethyl)-2-nitro-imino-5-benzyl-3-methyl-1,3,5-triazacyclohexan (95.7%ig nach ISTD) werden in 40 ml Acetonitril vorgelegt, und bei 0 bis 5°C wird gasförmiger Chlorwasserstoff bis zur Sättigung unter Rühren in die Suspension eingeleitet. Bei leichter Exothermie bleibt die Suspension bestehen. Man erwärmt auf 75°C, wobei Chlorwasserstoff entweicht, und nach 2 h Rühren bei dieser Temperatur hat sich eine klare Lösung gebildet. Man kühlt dann auf 0 bis -5°C. Es fällt ein Feststoff aus, man saugt ab, wäscht mit etwas Petrolether und erhält einen nahezu weißen Feststoff.

| | |
|---|---|
| Ausbeute: | 1.3 g (43.4% d. Th., 41.7 % Gehalt nach HPLC, 56.8% Ausgangsmaterial nach HPLC) |

Die Reinheits-/Gehaltsbestimmung in obigen Beispielen erfolgt nach Methoden der HPLC. Es kann mit und ohne internen Standard (ISTD) gearbeitet werden.

Analog obigen Beispielen können auch die in der folgenden Tabelle aufgeführten Verbindungen der Formel (I) erhalten werden:

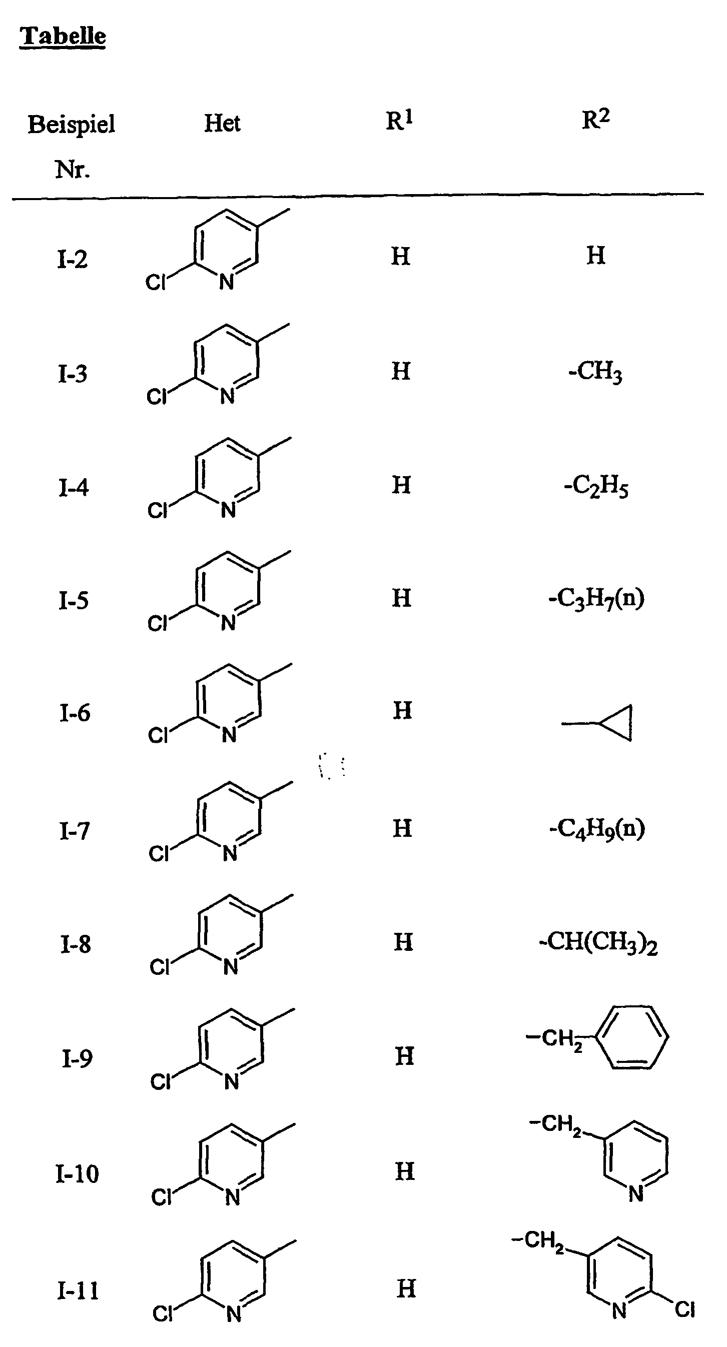

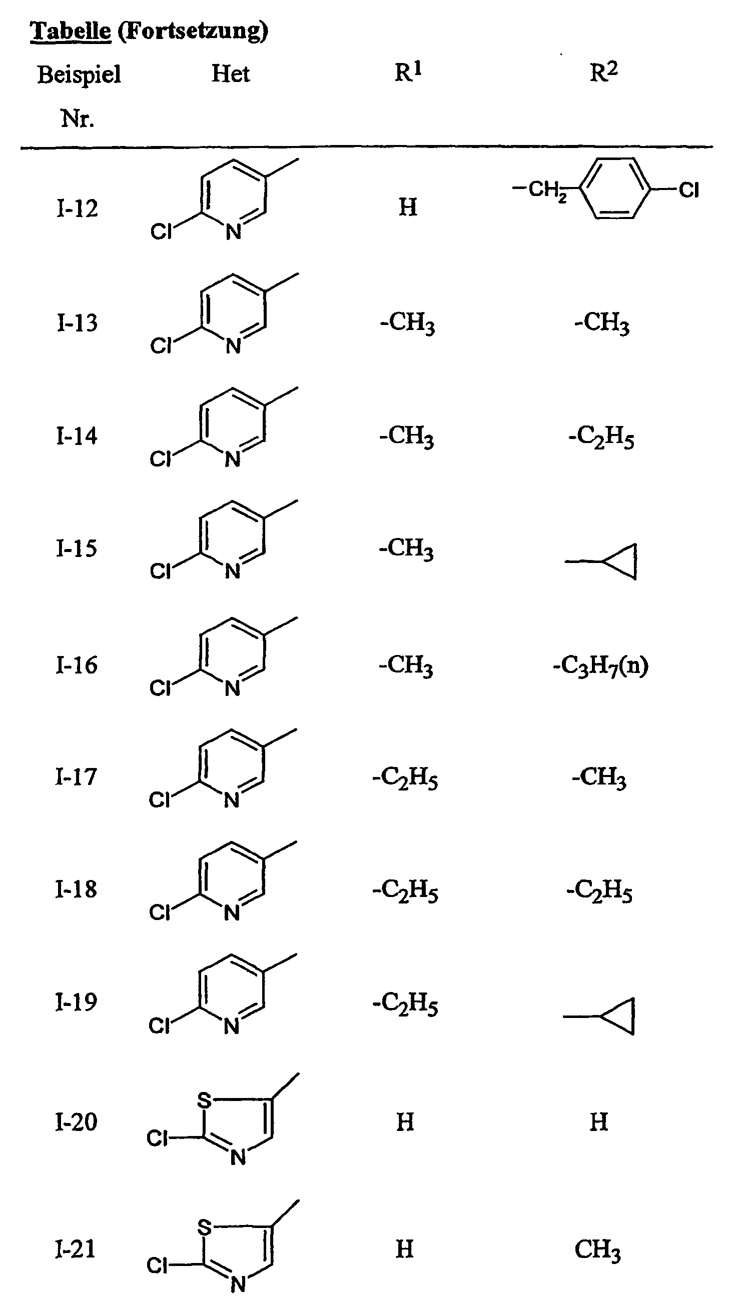

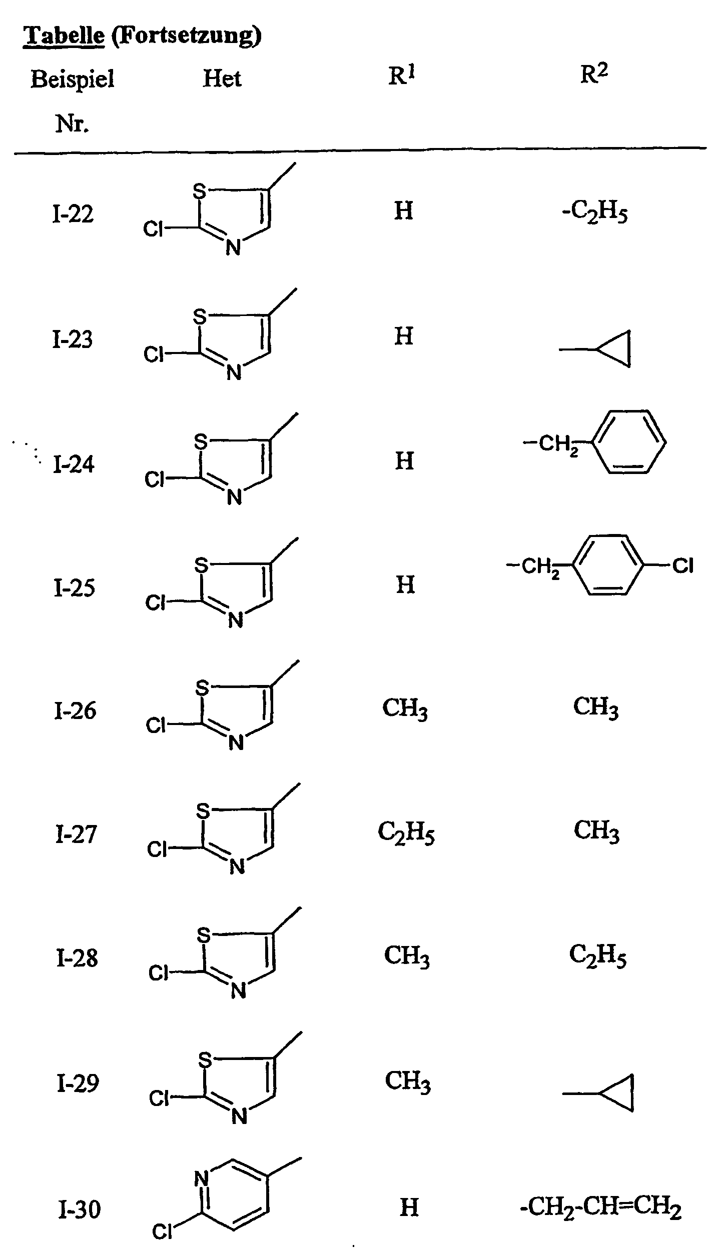

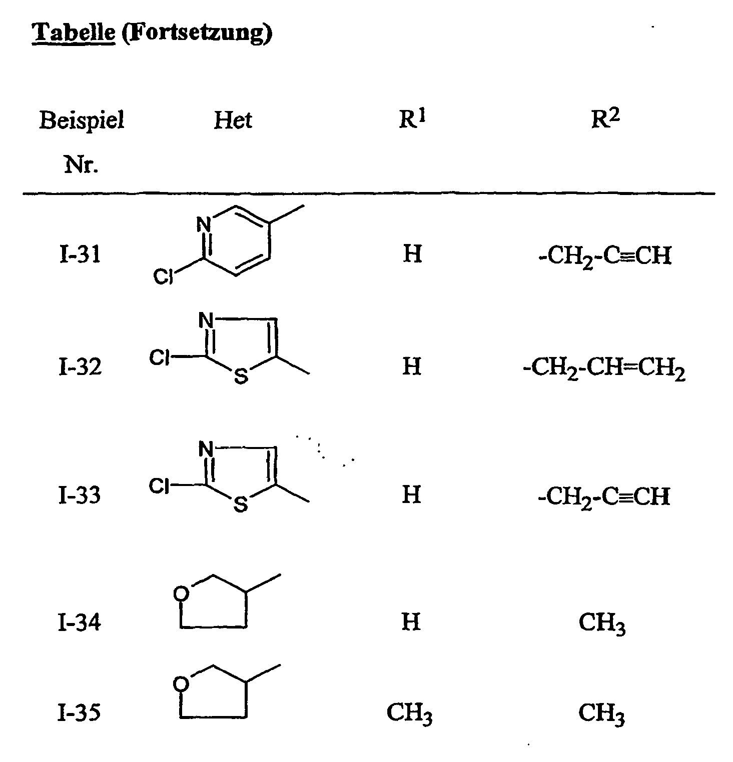

## Patentansprüche

1. Verfahren zur Herstellung von Verbindungen der Formel (I) 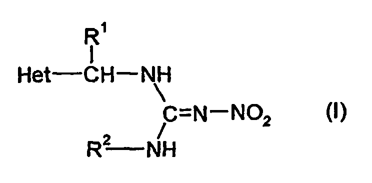 worin
R¹ für Wasserstoff oder C₁-C₄-Alkyl steht,
R² für Wasserstoff, C₁-C₆-Alkyl, C₃-C₆-Cycloalkyl oder einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen; durch C₁-C₃₋Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl; durch ein bis zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃₋Alkenyl, C₂-C₃-Alkinyl, C₁-C₃-Alkoxy, C₂-C₃-Halogenalkenyl mit 1 bis 5 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Halogen, Cyano oder Nitro substituiertes 5-Thiazolyl; oder durch ein bis vier (bevorzugt ein oder zwei) Reste aus der Gruppe C₁-C₃₋Halogenalkyl mit 1 bis 7 Halogenatomen, Cyclopropyl, Halogencyclopropyl mit 1 bis 3 Halogenatomen, C₂-C₃-Alkenyl, C₂₋C₃-Alkinyl, C₂-C₃-Halogenalkenyl mit 1 bis 5 Halogenatomen, C₂₋C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃-Alkoxy oder Halogen substituiertes 3-Pyridyl steht,
Het für einen unsubstituierten oder substituierten aromatischen oder nicht-aromatischen, monocyclischen oder bicyclischen heterocyclischen Rest bevorzugt aus der Reihe 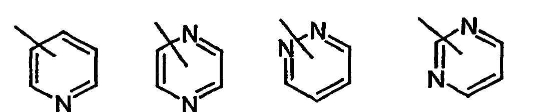 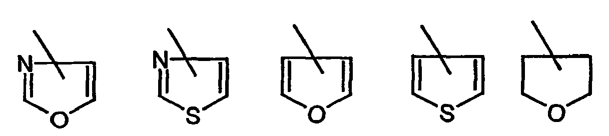 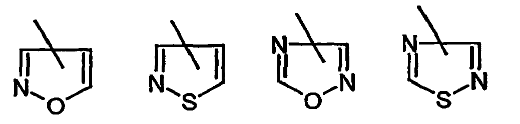 steht, der ein bis zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃₋Halogenalkenyl mit 1 bis 5 Halogenatomen, C₂-C₃-Halogenallcinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃-Alkoxy und Halogen enthalten kann,
**dadurch gekennzeichnet, dass** man eine Verbindung der Formel (II) 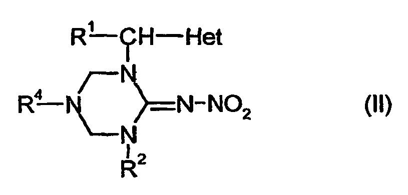 worin
R¹, R² und Het die oben angegebenen Bedeutungen haben und
R⁴ für jeweils unsubsituiertes oder substituiertes C₁-C₁₀-Alkyl, C₃-C₆₋Cycloalkyl, Phenyl, Arylalkyl oder Heterocyclylmethyl steht, wobei Heterocyclyl für einen ungesättigten oder gesättigten 5- oder 6-gliedrigen Heterocyclus mit einem oder mehreren Heteroatomen aus der Reihe Stickstoff, Sauerstoff und Schwefel wie z.B. Furan, Tetrahydrofinran, Thiophen oder Pyridin steht,
in Gegenwart eines Nitrils mit 3 bis 5 Kohlenstoffatomen mit wasserfreiem Halogenwasserstoff umsetzt.

2. Verfahren gemäß Anspruch 1, **dadurch gekennzeichnet, dass** man eine Verbindung der Formel (II) 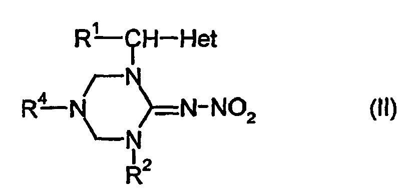 in welcher
R¹ für Wasserstoff, Methyl, Ethyl, n- oder i-Propyl steht,
R² für Wasserstoff, Methyl, Ethyl, n-Propyl, i-Propyl oder n-Butyl, Cyclopropyl, Cyclopentyl, Cyclohexyl oder für einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen; durch C₁-C₃₋Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl; durch ein bis zwei (bevorzugt einen) Substituenten aus der Grippe C₁-C₃-Alkyl, C₁-C₃- Halogenalkyl mit 1 bis 7 Halogenatomen, Cyclopropyl, Halogencyclopropyl mit 1 bis 3 Halogenatomen, C₂-C₃₋Alkenyl, C₂-C₃-Alkinyl, C₁-C₃-Alkoxy, C₂-C₃-Halogenalkenyl mit 1 bis 5 Halogenatomen, C₂-C₃-HaIogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Halogen, Cyano oder Nitro substituiertes 5-Thiazolyl; oder durch ein bis zwei (bevorzugt einen) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃₋Halogenalkenyl mit 1 bis 5 Halogenatomen, C₂-C₃-Halogenalkinyl mit 1 bis 3 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy mit 1 bis 5 Halogenatomen, Halogenallylthio mit 1 bis 5 Halogenatomen, Cyano, Nitro, C₁-C₃-Alkyl, C₁-C₃-Alkoxy oder Halogen substituiertes 3-Pyridyl steht,
R⁴ für unsubstituiertes oder durch 1 bis 6 Reste aus der Gruppe Halogen, Hydroxy, C₁-C₄-Alkoxy, C₁-C₄-Halogenalkoxy mit 1 bis 9 Halogenatomen, Di-(C₁-C₄-allcyl)-amino oder C₁-C₅-Alkoxycarbonyl substituiertes C₁-C₁₀-Alkyl; für unsubstituiertes oder durch 1 bis 4 Reste aus der Reihe C₁-C₄-Alkyl oder Halogen substituiertes C₃-C₆-Cycloalkyl; für jeweils unsubstituiertes oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro oder Cyano substituiertes Phenyl, Benzyl oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht,
Het für einen unsubstituierten oder einfach oder zweifach (bevorzugt einfach) substituierten heterocyclischen Rest aus der Reihe 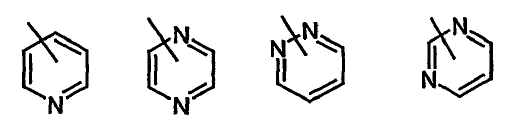 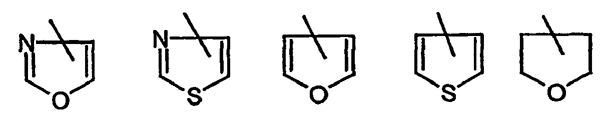 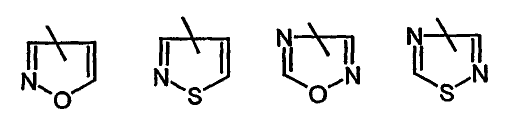 steht, insbesondere für 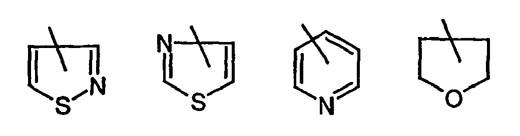 wobei die Substituenten aus der Reihe Fluor, Chlor, Brom, Methyl, Ethyl, Methoxy und Ethoxy ausgewählt sind,
einsetzt.

3. Verfahren gemäß Anspruch 1, **dadurch gekennzeichnet, dass** man eine Verbindung der Formel (II) 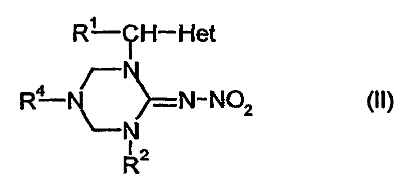 in welcher
R¹ für Wasserstoff, Methyl oder Ethyl steht,
R² für Wasserstoff, Methyl, Ethyl, n-Propyl, i-Propyl, n-Butyl, Cyclopropyl, Cyclopentyl, Cyclohexyl oder für einen Rest -CH₂R³ steht,
R³ für C₂-C₃-Alkenyl, C₂-C₃-Alkinyl, Phenyl, Cyanophenyl, Nitrophenyl, Halogenphenyl mit 1 bis 3 Halogenatomen; durch C₁-C₃₋Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl; jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenallcylthio mit 1 bis 7 Halogenatomen, Halogen, Cyano oder Nitro substituiertes 5-Thiazolyl oder 3-Pyridyl steht,
R⁴ für C₁-C₁₀-Alkyl; für durch 1 bis 6 Reste aus der Gruppe Halogen, C₁-C₄-Alkoxy und C₁-C₄-Halogenalkoxy mit 1 bis 9 Halogenatomen substituiertes C₁-C₈-Alkyl; für unsubstituiertes oder durch 1 oder 2 Reste aus der Reihe Methyl, Ethyl, Fluor und Chlor substituiertes C₃₋C₆-Cycloalkyl; für unsubstituiertes oder jeweils durch 1 bis 3 Ringsubstituenten aus der Gruppe Halogen, C₁-C₄-Alkyl, C₁-C₄₋Halogenalkyl mit 1 bis 9 Halogenatomen, C₁-C₄-Alkoxy, C₁-C₄₋Halogenalkoxy mit 1 bis 9 Halogenatomen, C₁-C₄-Alkylthio, Nitro oder Cyano substituiertes Phenyl, Benzyl oder Heterocyclylmethyl, wobei Heterocyclyl für einen ungesättigten oder gesättigten 5- oder 6-gliedrigen Heterocyclus mit einem Heteroatom aus der Reihe Stickstoff, Sauerstoff und Schwefel wie z.B. Furan, Tetrahydrofuran, Thiophen oder Pyridin steht,
Het für jeweils unsubstituiertes oder einfach oder zweifach (insbesondere einfach) substituiertes Thiazolyl, Pyridyl oder Tetrahydrofuranyl steht, wobei die Substituenten aus der Reihe Fluor, Chlor, Methyl und Methoxy ausgewählt sind,
einsetzt.

4. Verfahren gemäß Anspruch 1, **dadurch gekennzeichnet, dass** man eine Verbindung der Formel (II) 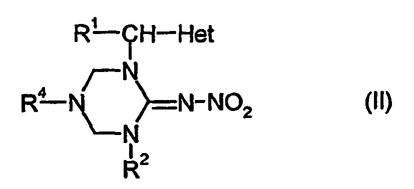 in welcher
R¹ für Wasserstoff, Methyl oder Ethyl, hervorgehoben für Wasserstoff steht,
R² für Wasserstoff, Methyl, Ethyl, n-Propyl, i-Propyl, n-Butyl, Cyclopropyl, Cyclopentyl, Allyl, Propargyl, Benzyl, p-Chlorbenzyl, 3-Pyridylmethyl oder 6-Chlor-3-pyridylmethyl steht,
R⁴ für Methyl, Ethyl, n-Propyl, Cyclopropyl, Cyclopentyl, Cyclohexyl, Phenyl, Benzyl oder Furfuryl steht,
Het für einen der Reste 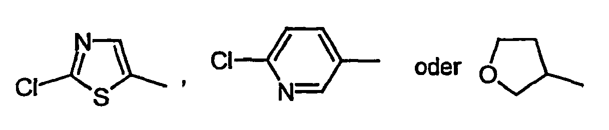 steht,
einsetzt.

5. Verfahren gemäß Anspruch 1, **dadurch gekennzeichnet, dass** man eine Verbindung der Formel (IIa) 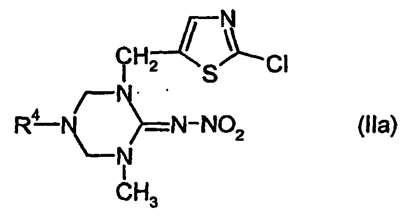 in welcher
R⁴ die in Anspruch 1 angegebenen Bedeutungen hat,
einsetzt.

6. Verfahren gemäß Anspruch 1, **dadurch gekennzeichnet, dass** man eine Verbindung der Formel (IIb) 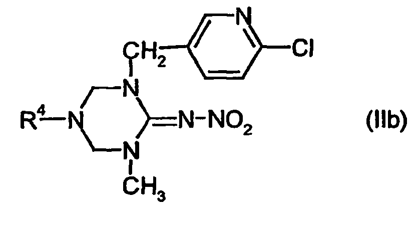 in welcher
R⁴ die in Anspruch 1 angegebenen Bedeutungen hat,
einsetzt.

7. Verfahren gemäß Anspruch 1, **dadurch gekennzeichnet, dass** man eine Verbindung der Formel (IIc) 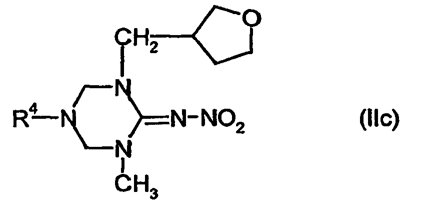 in welcher
R⁴ die in Anspruch 1 angegebenen Bedeutungen hat,
einsetzt.

8. Verfahren gemäß Anspruch 1, **dadurch gekennzeichnet, dass** die Umsetzung in Gegenwart eines aliphatischen Mono- oder Dinitrils mit 3 bis 5 Kohlenstoffatomen durchgeführt wird.

9. Verfahren gemäß Anspruch 8, **dadurch gekennzeichnet, dass** das verwendete Nitril aus der Reihe Propionitril, Butyronitril, Valeronitril, Malonsäuredinitril (Malononitril), Bernsteinsäuredinitril (Succinonitril), Glutarsäuredinitril (Glutaronitril) ausgewählt ist.

10. Verfahren gemäß Anspruch 9, **dadurch gekennzeichnet, dass** das verwendete Nitril Propionitril oder Butyronitril ist.

11. Verfahren gemäß Anspruch 10, **dadurch gekennzeichnet, dass** das verwendete Nitril Butyronitril ist.

12. Verfahren gemäß Anspruch 1, **dadurch gekennzeichnet, dass** als wasserfreier Halogenwasserstoff Chlorwasserstoff, Bromwasserstoff oder Iodwasserstoff eingesetzt wird.

13. Verfahren gemäß Anspruch 12, **dadurch gekennzeichnet, dass** als wasserfreier Halogenwasserstoff Chlorwasserstoff oder Bromwasserstoff eingesetzt wird.

14. Verfahren gemäß Anspruch 13, **dadurch gekennzeichnet, dass** als wasserfreier Halogenwasserstoff Chlorwasserstoff eingesetzt wird.

15. Verfahren gemäß Anspruch 1, **dadurch gekennzeichnet, dass** es bei einer Temperatur zwischen -10°C und 200°C durchgeführt wird.

## Claims

1. Process for the preparation of compounds of the formula (I) 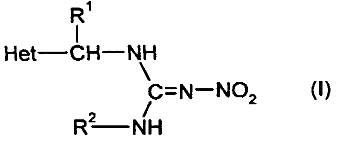 in which
R¹ is hydrogen or C₁-C₄-alkyl,
R² is hydrogen, C₁-C₆-alkyl, C₃-C₆-cycloalkyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms; phenyl substituted by C₁-C₃-alkyl, C₁-C₃-halogenoalkoxy having from 1 to 7 halogen atoms, C₁-C₃-alkoxy or C₁-C₃-halogenoalkoxy having from 1 to 7 halogen atoms; 5-thiazolyl substituted by one to two (preferably one) substituents from the group consisting of C₁-C₃₋alkyl, C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₁-C₃-alkoxy, C₂-C₃₋halogenoalkenyl having from 1 to 5 halogen atoms, C₂-C₃₋halogenoalkinyl having from 1 to 3 halogen atoms, C₁-C₃- halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy having from 1 to 5 halogen atoms, halogenoallylthio having from 1 to 5 halogen atoms, halogen, cyano or nitro; or 3-pyridyl substituted by one to four (preferably one or two) radicals from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃₋halogenoalkenyl having from 1 to 5 halogen atoms, C₂-C₃₋halogenoalkinyl having from 1 to 3 halogen atoms, C₁-C₃₋halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy having from 1 to 5 halogen atoms, halogenoallylthio having from 1 to 5 halogen atoms, cyano, nitro, C₁-C₃-alkyl, C₁-C₃-alkoxy or halogen,
Het is an unsubstituted or substituted aromatic or non-aromatic, monocyclic or bicyclic heterocyclic radical, preferably from the series 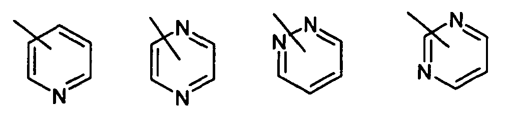 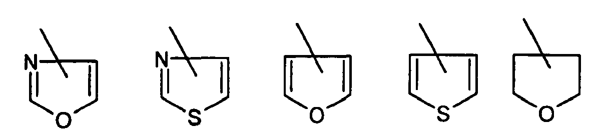 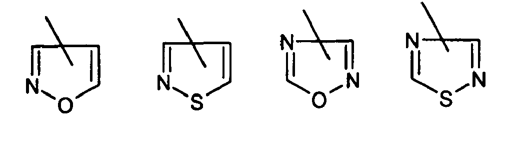 which may include one to two substituents from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃₋halogenoalkenyl having from 1 to 5 halogen atoms, C₂-C₃₋halogenoalkinyl having from 1 to 3 halogen atoms, C₁-C₃₋halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy having from 1 to 5 halogen atoms, halogenoallylthio having from 1 to 5 halogen atoms, cyano, nitro, C₁-C₃-alkyl, C₁-C₃-alkoxy and halogen,
**characterized in that** a compound of the formula (II) 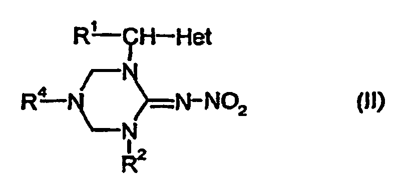 in which
R¹, R² and Het are as defined above, and
R⁴ is C₁-C₁₀-alkyl, C₃-C₆-cycloalkyl, phenyl, arylalkyl or heterocyclylmethyl, each of which may be unsubstituted or substituted, where heterocyclyl is an unsaturated or saturated 5- or 6-membered heterocycle containing one or more heteroatoms from the series nitrogen, oxygen and sulphur, such as, for example, furan, tetrahydrofuran, thiophene or pyridine,
in the presence of a nitrile having from 3 to 5 carbon atoms, is reacted with anhydrous hydrogen halide.

2. Process according to Claim 1, **characterized in that** a compound of the formula (II) 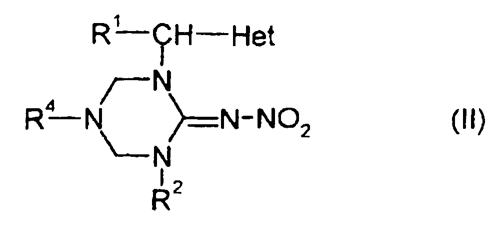 in which
R¹ is hydrogen, methyl, ethyl, n- or i-propyl,
R² is hydrogen, methyl, ethyl, n-propyl, i-propyl or n-butyl, cyclopropyl, cyclopentyl, cyclohexyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms; phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms; 5-thiazolyl substituted by one to two (preferably one) substituents from the group consisting of C₁-C₃₋alkyl, C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₁-C₃-alkoxy, C₂-C₃₋halogenoalkenyl having from 1 to 5 halogen atoms, C₂-C₃₋halogenoalkinyl having from 1 to 3 halogen atoms, C₁-C₃₋halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy having from 1 to 5 halogen atoms, halogenoallylthio having from 1 to 5 halogen atoms, halogen, cyano or nitro; or 3-pyridyl substituted by one to two (preferably one) radicals from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃-halogenoalkenyl having from 1 to 5 halogen atoms, C₂-C₃-halogenoalkinyl having from 1 to 3 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy having from 1 to 5 halogen atoms, halogenoallylthio having from 1 to 5 halogen atoms, cyano, nitro, C₁-C₃-alkyl, C₁-C₃-alkoxy or halogen,
R⁴ is unsubstituted C₁-C₁₀-alkyl or C₁-C₁₀-alkyl substituted by from 1 to 6 radicals from the group consisting of halogen, hydroxyl, C₁-C₄₋alkoxy, C₁-C₄-halogenoalkoxy having from 1 to 9 halogen atoms, di-(C₁-C₄-alkyl)-amino or C₁-C₅-alkoxycarbonyl; unsubstituted C₃₋C₆-cycloalkyl or C₃-C₆-cycloalkyl substituted by from 1 to 4 radicals from the series C₁-C₄-alkyl or halogen; in each case unsubstituted phenyl, benzyl or heterocyclylmethyl, or phenyl, benzyl or heterocyclylmethyl each substituted by from 1 to 3 ring substituents from the group consisting of halogen, C₁-C₄-alkyl, C₁₋C₄-halogenoalkyl having from 1 to 9 halogen atoms, C₁-C₄-alkoxy, C₁-C₄-halogenoalkoxy having from 1 to 9 halogen atoms, C₁-C₄₋alkylthio, nitro or cyano, where heterocyclyl is an unsaturated or saturated 5- or 6-membered heterocycle having one or two (preferably one) heteroatoms from the series nitrogen, oxygen and sulphur, such as, for example, furan, tetrahydrofuran, thiophene or pyridine,
Het is an unsubstituted or mono- or disubstituted (preferably monosubstituted) heterocyclic radical from the series 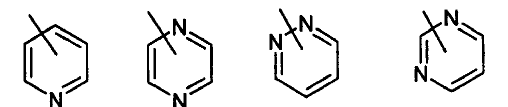 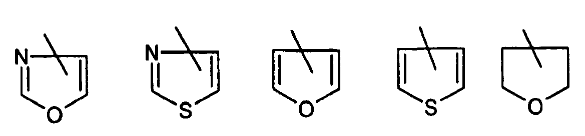 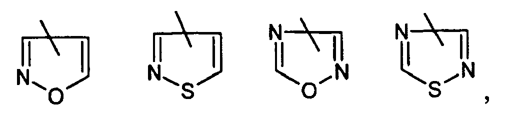 in particular 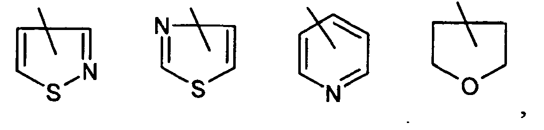 the substituents being chosen from the series fluorine, chlorine, bromine, methyl, ethyl, methoxy and ethoxy,
is used.

3. Process according to Claim 1, **characterized in that** a compound of the formula (II) 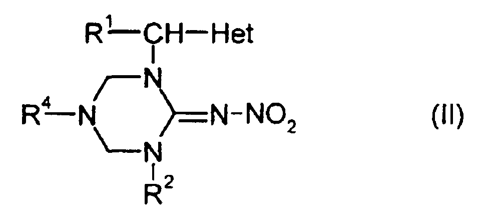 in which
R¹ is hydrogen, methyl or ethyl,
R² is hydrogen, methyl, ethyl, n-propyl, i-propyl, n-butyl, cyclopropyl, cyclopentyl, cyclohexyl or a radical -CH₂R³,
R³ is C₂-C₃-alkenyl, C₂-C₃-alkinyl, phenyl, cyanophenyl, nitrophenyl, halogenophenyl having from 1 to 3 halogen atoms; phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms; 5-thiazolyl or 3-pyridyl each substituted by one or two (preferably one) substituents from the group consisting of C₁-C₃-alkyl, C₁-C₃-halogenoalkyl having from 1 to 7 halogen atoms, C₁-C₃-alkoxy, C₁-C₃-halogenoalkoxy having from 1 to 7 halogen atoms, C₁-C₃-alkylthio, C₁-C₃-halogenoalkylthio having from 1 to 7 halogen atoms, halogen, cyano or nitro,
R⁴ is C₁-C₁₀-alkyl; C₁-C₈-alkyl, substituted by from 1 to 6 radicals from the group consisting of halogen, C₁-C₄-alkoxy and C₁-C₄₋halogenoalkoxy having from 1 to 9 halogen atoms; unsubstituted C₃-C₆-cycloalkyl or C₃-C₆-cycloalkyl substituted by 1 or 2 radicals from the series methyl, ethyl, fluorine and chlorine; unsubstituted phenyl, benzyl or heterocyclylmethyl, or phenyl, benzyl or heterocyclylmethyl each substituted by from 1 to 3 ring substituents from the group consisting of halogen, C₁-C₄-alkyl, C₁-C₄₋halogenoalkyl having from 1 to 9 halogen atoms, C₁-C₄-alkoxy, C₁-C₄-halogenoalkoxy having from 1 to 9 halogen atoms, C₁-C₄₋alkylthio, nitro or cyano, where heterocyclyl is an unsaturated or saturated 5- or 6-membered heterocycle containing one heteroatom from the series nitrogen, oxygen and sulphur, such as, for example, furan, tetrahydrofuran, thiophene or pyridine,
Het is thiazolyl, pyridyl or tetrahydrofuranyl, each of which may be unsubstituted or mono- or disubstituted (in particular monosubstituted), the substituents being chosen from the series fluorine, chlorine, methyl and methoxy,
is used.

4. Process according to Claim 1, **characterized in that** a compound of the formula (II) 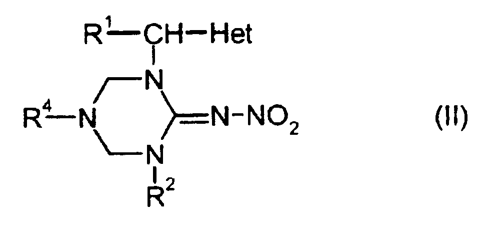 in which
R¹ is hydrogen, methyl or ethyl, especially hydrogen,
R² is hydrogen, methyl, ethyl, n-propyl, i-propyl, n-butyl, cyclopropyl, cyclopentyl, allyl, propargyl, benzyl, p-chlorobenzyl, 3-pyridylmethyl or 6-chloro-3-pyridylmethyl,
R⁴ is methyl, ethyl, n-propyl, cyclopropyl, cyclopentyl, cyclohexyl, phenyl, benzyl or furfuryl,
Het is one of the radicals 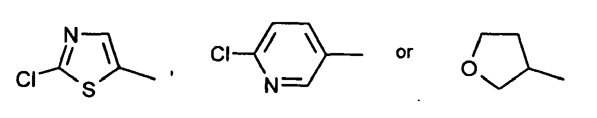
is used.

5. Process according to Claim 1, **characterized in that** a compound of the formula (IIa) 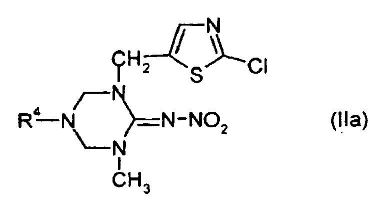 in which
R⁴ is as defined in Claim 1
is used.

6. Process according to Claim 1, **characterized in that** a compound of the formula (IIb) 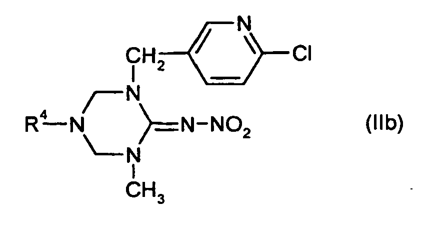 in which
R⁴ is as defined in Claim 1
is used.

7. Process according to Claim 1, **characterized in that** a compound of the formula (IIc) 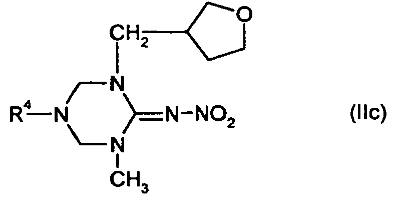 in which
R⁴ is as defined in Claim 1
is used.

8. Process according to Claim 1, **characterized in that** the reaction is carried out in the presence of an aliphatic mono- or dinitrile having 3 to 5 carbon atoms.

9. Process according to Claim 8, **characterized in that** the nitrile used is chosen from the series propionitrile, butyronitrile, valeronitrile, malononitrile, succinonitrile and glutaronitrile.

10. Process according to Claim 9, **characterized in that** the nitrile used is propionitrile or butyronitrile.

11. Process according to Claim 10, **characterized in that** the nitrile used is butyronitrile.

12. Process according to Claim 1, **characterized in that** the anhydrous hydrogen halide used is hydrogen chloride, hydrogen bromide or hydrogen iodide.

13. Process according to Claim 12, **characterized in that** the anhydrous halogen halide used is hydrogen chloride or hydrogen bromide.

14. Process according to Claim 13, **characterized in that** the anhydrous hydrogen halide used is hydrogen chloride.

15. Process according to Claim 1, **characterized in that** it is carried out at a temperature between -10°C and 200°C.

## Revendications

1. Procédé de fabrication de composés de la formule (I) 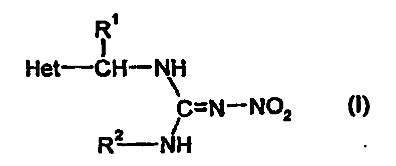 où
R¹ représente l'hydrogène ou un groupe alkyle en C₁-C₄,
R² représente l'hydrogène, un groupe alkyle en C₁-C₆, un groupe cycloalkyle en C₃-C₆ ou un reste -CH₂R³,
R³ représente un groupe alcényle en C₂-C₅, un groupe alcynyle en C₂-C₅, un groupe phényle, un groupe cyanophényle, un groupe nitrophényle, un groupe halogénophényle avec 1 à 3 atomes d'halogène; un groupe phényle substitué par un groupe alkyle en C₁-C₃, un groupe halogénoalkyle en C₁-C₃ avec 1 à 7 atomes d' halogène, un groupe alcoxy en C₁-C₃ ou halogénoalcoxy en C₁-C₃ avec 1 à 7 atomes d'halogène; un groupe 5-thiazolyle substitué par un à deux (de préférence un) substituants parmi les groupes alkyle en C₁-C₃, halogénoalkyle en C₁-C₃ avec 1 à 7 atomes d'halogène, cyclopropyle, halogénocyclopropyle avec 1 à 3 atomes d' halogène, alcényle en C₂-C₃. alcynyle en C₂-C₃, alcoxy en C₁-C₃, halogénoalkyle en C₂-C₃ avec 1 à 5 atomes d'halogène, halogénoalcynyle en C₂-C₃ avec 1 à 3 atomes d'halogène, halogénoalcoxy en C₁-C₃ avec 1 à 7 atomes d'halogène, alkylthio en C₁-C₃, halogénoalkylthio en C₁-C₃ avec 1 à 7 atomes d'halogène, allyloxy, propargyloxy, allylthio, propargylthio, halogénoallyloxy avec 1 à 5 atomes d'halogène, halogénoallylthio avec 1 à 5 atomes d'halogène, halogène, cyano ou nitro; ou un groupe 3-pyridyle substitué par un à quatre (de préférence un ou deux) restes parmi les groupes halogénoalkyle en C₁-C₃ avec 1 à 7 atomes d'halogène, cyclopropyle, halogénocyclopropyle avec 1 à 3 atomes d'halogène, alcényle en C₂-C₃, alcynyle en C₂-C₃, halogénoalkyle en C₂-C₃ avec 1 à 5 atomes d'halogène, halogénoalcynyle en C₂-C₃ avec 1 à 3 atomes d'halogène, halogénoalcoxy en C₁-C₃ avec 1 à 7 atomes d'halogène, alkylthio en C₁-C₃, halogénoalkylthio en C₁-C₃ avec 1 à 7 atomes d'halogène, allyloxy, propargyloxy, allylthio, propargylthio, halogénoallyloxy avec 1 à 5 atomes d'halogène, halogénoallylthio avec 1 à 5 atomes d'halogène, cyano, nitro, alkyle en C₁-C₃, alcoxy en C₁-C₃ ou halogène,
Het représente un reste hétérocyclique monocyclique ou bicyclique, aromatique ou non aromatique, non substitué ou substitué, de préférence de la série 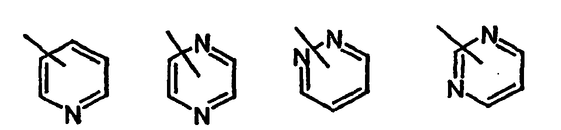 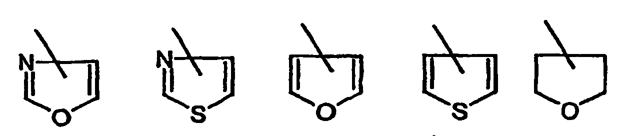 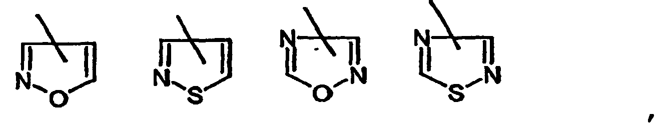 qui peut contenir un ou deux substituants parmi les groupes halogénoalkyle en C₁-C₃ avec 1 à 7 atomes d'halogène, cyclopropyle, halogénocyclopropyle avec 1 à 3 atomes d' halogène, alcényle en C₂-C₃, alcynyle en C₂-C₃, halogénoalkyle en C₂-C₃ avec 1 à 5 atomes d'halogène, halogénoalcynyle en C₂-C₃ avec 1 à 3 atomes d'halogène, halogénoalcoxy en C₁-C₃ avec 1 à 7 atomes d'halogène, alkylthio en C₁-C₃, halogénoalkylthio en C₁-C₃ avec 1 à 7 atomes d'halogène, allyloxy, propargyloxy, allylthio, propargylthio, halogénoallyloxy avec 1 à 5 atomes d'halogène, halogénoallylthio avec 1 à 5 atomes d'halogène, cyano, nitro, alkyle en C₁-C₃, alcoxy en C₁-C₃ et halogène,
**caractérisé en ce qu'**on fait réagir un composé de la formule (II) 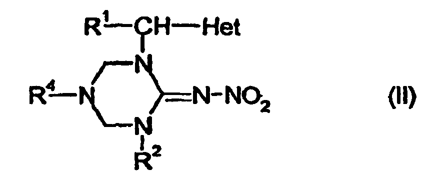 où
R¹, R² et Het ont les significations données ci-dessus et
R⁴ représente un groupe, respectivement non substitué ou substitué, alkyle en C₁-C₁₀, cycloalkyle en C₃₋C₆, phényle, arylalkyle ou hétérocyclylméthyle, hétérocyclyl désignant un hétérocycle à 5 ou 6 éléments, non saturé ou saturé, avec un ou plusieurs hétéroatomes de la série de l'azote, de l'oxygène et du soufre, tels que par exemple le furanne, le tétrahydrofuranne, le thiophène ou la pyridine,
avec un hydracide anhydre en présence d'un nitrile avec 3 à 5 atomes de carbone.

2. Procédé suivant la revendication 1, **caractérisé en ce qu'**on fait réagir un composé de la formule (II) 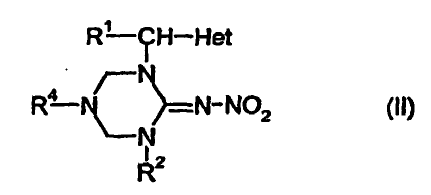 dans laquelle
R¹ représente l'hydrogène, un groupe méthyle, éthyle, n-propyle ou isopropyle,
R² représente l'hydrogène, un groupe méthyle, éthyle, n-propyle, isopropyle ou n-butyle, un groupe cyclopropyle, cyclopentyle, cyclohexyle ou un reste -CH₂R³,
R³ représente un groupe alcényle en C₂-C₅, un groupe alcynyle en C₂-C₅, un groupe phényle, un groupe cyanophényle, un groupe nitrophényle, un groupe halogénophényle avec 1 à 3 atomes d'halogène; un groupe phényle substitué par un groupe alkyle en C₁-C₃, un groupe halogénoalkyle en C₁-C₃ avec 1 à 7 atomes d'halogène, un groupe alcoxy en C₁-C₃ ou halogénoalcoxy en C₁-C₃ avec 1 à 7 atomes d'halogène; un groupe 5-thiazolyle substitué par un à deux (de préférence un) substituants parmi les groupes alkyle en C₁-C₃, halogénoalkyle en C₁-C₃ avec 1 à 7 atomes d'halogène, cyclopropyle, halogénocyclopropyle avec 1 à 3 atomes d' halogène, alcényle en C₂-C₃, alcynyle en C₂-C₃, alcoxy en C₁-C₃, halogénoalkyle en C₂-C₃ avec 1 à 5 atomes d'halogène, halogénoalcynyle en C₂-C₃ avec 1 à 3 atomes d'halogène, halogénoalcoxy en C₁-C₃ avec 1 à 7 atomes d'halogène, alkylthio en C₁-C₃, halogénoalkylthio en C₁-C₃ avec 1 à 7 atomes d'halogène, allyloxy, propargyloxy, allylthio, propargylthio, halogénoallyloxy avec 1 à 5 atomes d'halogène, halogénoallylthio avec 1 à 5 atomes d'halogène, halogène, cyano ou nitro; ou un groupe 3-pyridyle substitué par un à deux (de préférence un) restes parmi les groupes halogénoalkyle en C₁-C₃ avec 1 à 7 atomes d'halogène, cyclopropyle, halogénocyclopropyle avec 1 à 3 atomes d' halogène, alcényle en C₂-C₃, alcynyle en C₂-C₃, halogénoalkyle en C₂-C₃ avec 1 à 5 atomes d'halogène, halogénoalcynyle en C₂-C₃ avec 1 à 3 atomes d'halogène, halogénoalcoxy en C₁-C₃ avec 1 à 7 atomes d'halogène, alkylthio en C₁-C₃, halogénoalkylthio en C₁-C₃ avec 1 à 7 atomes d'halogène, allyloxy, propargyloxy, allylthio, propargylthio, halogénoallyloxy avec 1 à 5 atomes d'halogène, halogénoallylthio avec 1 à 5 atomes d'halogène, cyano, nitro, alcoxy en C₁-C₃ ou halogène,
R⁴ représente un groupe alkyle en C₁-C₁₀ non substitué ou substitué par 1 à 6 restes constitué d'un halogène, d'un groupe hydroxy, alcoxy en C₁-C₄, halogénoalcoxy en C₁-C₄ avec 1 à 9 atomes d'halogène, di(alkyle en C₁-C₄) amino ou alcoxycarbonyle en C₁-C₅; un groupe cycloalkyle en C₃-C₆ non substitué ou substitué par 1 à 4 restes de la série alkyle en C₁-C₄ ou halogène; un groupe phényle, benzyle ou hétérocyclylméthyle non substitué ou substitué respectivement par 1 à 3 substituants du cycle constitués d'un halogène, d'un groupe alkyle en C₁-C₄, halogénoalkyle en C₁-C₄ avec 1 à 9 atomes d'halogène, alcoxy en C₁-C₄, halogénoalcoxy en C₁-C₄ avec 1 à 9 atomes d'halogène, alkylthio en C₁-C₄, nitro ou cyano, hétérocyclyl désignant un hétérocycle à 5 ou 6 éléments, non saturé ou saturé, avec un ou deux hétéroatomes (de préférence un) de la série de l'azote, de l'oxygène et du soufre, tels que par exemple le furanne, le tétrahydrofuranne, le thiophène ou la pyridine,
Het représente un reste hétérocyclique non substitué ou substitué une fois ou deux fois (de préférence une fois) de la série 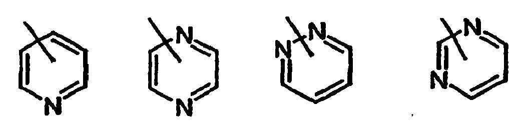 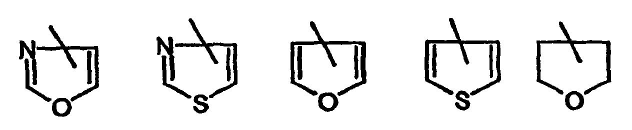 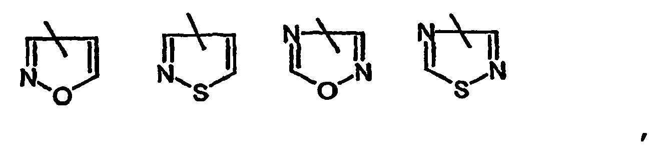 en particulier 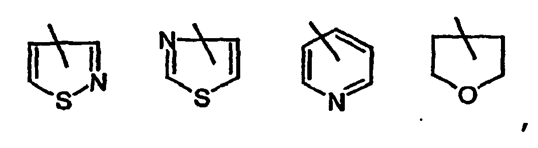 les substituants étant sélectionnés dans la série du fluor, du chlore, du brome, des groupes méthyle, éthyle, méthoxy et éthoxy.

3. Procédé suivant la revendication 1, **caractérisé en ce qu'**on fait réagir un composé de la formule (II) 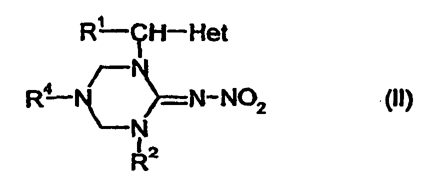 dans laquelle
R¹ représente l'hydrogène, un groupe méthyle ou éthyle,
R² représente l'hydrogène, un groupe méthyle, éthyle, n-propyle, isopropyle, n-butyle, cyclopropyle, cyclopentyle, cyclohexyle ou un reste -CH₂R³,
R³ représente un groupe alcényle en C₂-C₃, un groupe alcynyle en C₂-C₃, un groupe phényle, un groupe cyanophényle, un groupe nitrophényle, un groupe halogénophényle avec 1 à 3 atomes d'halogène; un groupe phényle substitué par un groupe alkyle en C₁-C₃, un groupe halogénoalkyle en C₁-C₃ avec 1 à 7 atomes d'halogène, un groupe alcoxy en C₁-C₃ ou halogénoalcoxy en C₁-C₃ avec 1 à 7 atomes d'halogène; un groupe 5-thiazolyle ou 3-pyridyle, substitué respectivement par un ou deux (de préférence un) substituant parmi les groupes alkyle en C₁-C₃, halogénoalkyle en C₁-C₃ avec 1 à 7 atomes d'halogène, alcoxy en C₁-C₃, halogénoalcoxy en C₁-C₃ avec 1 à 7 atomes d'halogène, alkylthio en C₁-C₃, halogénoalkylthio en C₁-C₃ avec 1 à 7 atomes d'halogène, halogène, cyano ou nitro,
R⁴ représente un groupe alkyle en C₁-C₁₀; un groupe alkyle en C₁-C₈ substitué par 1 à 6 restes constitués d'un halogène, d'un groupe alcoxy en C₁-C₄ et halogénoalcoxy en C₁-C₄ avec 1 à 9 atomes d' halogène; un groupe cycloalkyle en C₃-C₆ non substitué ou substitué par 1 ou 2 restes de la série méthyle, éthyle, fluor et chlore; un groupe phényle, benzyle ou hétérocyclylméthyle non substitué ou substitué respectivement par 1 à 3 substituants du cycle constitués d'un halogène, d'un groupe alkyle en C₁-C₄, halogénoalkyle en C₁-C₄ avec 1 à 9 atomes d'halogène, alcoxy en C₁-C₄, halogénoalcoxy en C₁-C₄ avec 1 à 9 atomes d'halogène, alkylthio en C₁-C₄, nitro ou cyano, hétérocyclyl désignant un hétérocycle à 5 ou 6 éléments, non saturé ou saturé, avec un hétéroatome de la série de l'azote, de l'oxygène et du soufre, tels que par exemple le furanne, le tétrahydrofuranne, le thiophène ou la pyridine,
Het représente un groupe thiazolyle, pyridyle ou tétrahydrofurannyle, respectivement non substitué ou substitué une fois ou deux fois (en particulier une fois), les substituants étant sélectionnés dans la série fluor, chlore, méthyle et méthoxy.

4. Procédé suivant la revendication 1, **caractérisé en ce qu'**on fait réagir un composé de la formule (II) 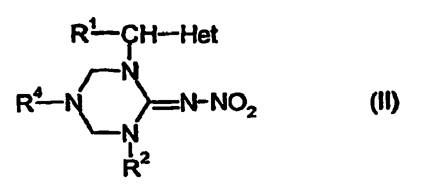 dans laquelle
R¹ représente l'hydrogène, un groupe méthyle ou éthyle, mis en évidence pour l'hydrogène,
R² représente l'hydrogène, un groupe méthyle, éthyle, n-propyle, isopropyle, n-butyle, cyclopropyle, cyclopentyle, allyle, propargyle, benzyle, p-chlorbenzyle, 3-pyridylméthyle ou 6-chloro-3-pyridylméthyle,
R⁴ représente un groupe méthyle, éthyle, n-propyle, cyclopropyle, cyclopentyle, cyclohexyle, phényle, benzyle ou furfuryle,
représente un des restes

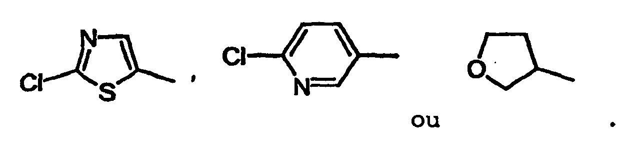

5. Procédé suivant la revendication 1, **caractérisé en ce qu'**on fait réagir un composé de la formule (IIa) 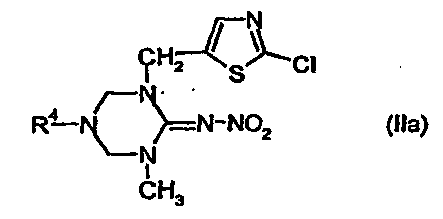 dans laquelle
R⁴ a les significations données dans la revendication 1.

6. Procédé suivant la revendication 1, **caractérisé en ce qu'**on fait réagir un composé de la formule (IIb) 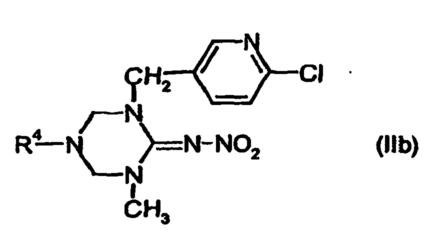 dans laquelle
R⁴ a les significations données dans la revendication 1.

7. Procédé suivant la revendication 1, **caractérisé en ce qu'**on fait réagir un composé de la formule (IIc) 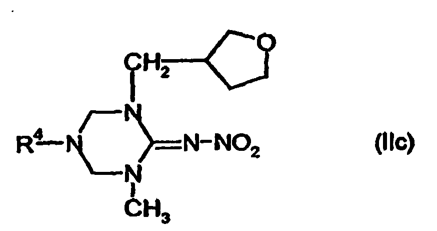 dans laquelle
R⁴ a les significations données dans la revendication 1.

8. Procédé suivant la revendication 1, **caractérisé en ce que** la réaction est effectuée en présence d'un mononitrile ou dinitrile aliphatique avec 3 à 5 atomes de carbone.

9. Procédé suivant la revendication 8, **caractérisé en ce que** le nitrile utilisé est sélectionné parmi la série propionitrile, butyronitrile, valéronitrile, dinitrile d'acide maléique (malononitrile), nitrile d'acide succinique (succinonitrile), nitrile d'acide glutarique (glutaronitrile).

10. Procédé suivant la revendication 9, **caractérisé en ce que** le nitrile utilisé est du propionitrile ou du butyronitrile.

11. Procédé suivant la revendication 10, **caractérisé en ce que** le nitrile utilisé est du butyronitrile.

12. Procédé suivant la revendication 1, **caractérisé en ce qu'**on utilise comme hydracide anhydre du chlorure d'hydrogène, du bromure d'hydrogène ou de l'iodure d'hydrogène.

13. Procédé suivant la revendication 12, **caractérisé en ce qu'**on utilise comme hydracide anhydre du chlorure d'hydrogène ou du bromure d'hydrogène.

14. Procédé suivant la revendication 13, **caractérisé en ce qu'**on utilise comme hydracide anhydre du chlorure d'hydrogène.

15. Procédé suivant la revendication 1, **caractérisé en ce qu'**il est effectué à une température de entre -10°C et 200°C.
